# NEUE EUROPÄISCHE PATENTSCHRIFT

(11) **EP 0 738 137 B2**
(45) Veröffentlichungstag und Bekanntmachung des Hinweises auf die Entscheidung über den Einspruch: **04.02.2004**
(45) Hinweis auf die Patenterteilung: 06.09.2000
(21) Anmeldenummer: 95905035.2
(22) Anmeldetag: 04.01.1995
(51) Int. Cl.: A61K 6/083, A61C 19/00, A61C 3/08

(54) **DENTALWERKSTOFF, SOWIE WERKZEUG FÜR DESSEN VERWENDUNG**
DENTAL MATERIAL AND TOOL FOR ITS USE
MATERIAU DENTAIRE ET OUTIL POUR SON UTILISATION

(30) Priorität: 04.01.1994 DE 4400073
(43) Veröffentlichungstag der Anmeldung: 23.10.1996
(73) Patentinhaber: KREBBER, Burghardt, D-86899 Landsberg (DE)
(72) Erfinder: KREBBER, Burghardt, D-86899 Landsberg (DE)
(74) Vertreter: Wächtershäuser, Günter, Prof. Dr.
(86) Internationale Anmeldenummer: PCT/DE1995/000007
(87) Internationale Veröffentlichungsnummer: WO 1995/018597

(56) Entgegenhaltungen:
- EP-B- 0 726 739
- WO-A-89/04640
- DE-A- 3 819 777
- DE-A- 3 831 232
- DE-C- 4 219 793
- DE-U- 9 400 070
- US-A- 5 098 304
- US-A- 5 176 951
- US-A- 5 218 070
- Japanese patent n° 97625/1993 and its english translation
- Japanese patent n°1218357/1990 ans a partial english translation + english abstract thereof
- Catalog "General information" by Kulzer Corporation and partial english translation of page 3 thereof

## Beschreibung

Die Erfindung betrifft einen mit UV-Strahlen, Laserstrahlen oder Licht härtbaren Dentalwerkstoff für die Herstellung von Zahnfüllungen, Inlays/Onlays, Kronen, künstlichen Zähnen, Stiftzähnen, Brücken, Zahnprothesen und Implantaten. Weiterhin wird ein Werkzeug zur Polymerisation des erfindungsgemäßen Dentalwerkstoffs offenbart.

An den Zahnersatz werden besonders Forderungen an die Ungiftigkeit, Biokompatibilität, Bruchfestigkeit, Verschleißfestigkeit, ästhetischem Aussehen, Resistenz gegen chemische Einflüsse, wie durch Speichel und Speisen, sowie Resistenz gegen elektro-galvanische und elektromagnetische Wirkungen gestellt.

Bekannt und weit verbreitet sind Zahnfüllungen aus Amalgam. Sie sind billig und haben gute mechanische Eigenschaften. Wegen seines Quecksilbergehaltes ist Amalgam jedoch giftig und kann starke gesundheitliche Schäden hervorrufen. Ferner wirken sich elektro-galvanische und elektromagnetische Eigenschaften gesundheitsschädigend aus.

Ferner ist Gold als Zahnersatz bekannt. Es hat gute mechanische Eigenschaften. Gold ist sehr teuer und wird aus ästhetischen Gründen oft nicht akzeptiert. In Nachbarschaft mit anderen Metallen können gesundheitsschädigende elektrische Ströme fließen.

Ferner sind keramische Werkstoffe bekannt, die sich wegen ihres Aussehens und Abriebfestigkeit als Zahnersatz gut eignen. Sie besitzen, besonders bei dünnwandigen Teilen, ein hohes Frakturrisiko und lassen sich schwer bearbeiten.

Es sind auch Kombinationen von Gold und Keramik im Einsatz. Bekannt ist auch, daß aus Keramikoder Kunststoffblöcken Inlays/Onlays gefräst werden. Diese Einsätze bergen ein hohes Frakturrisiko und die Okklusion ist ungenau. Die Maschine ist sehr teuer.

Weiter sind Kompositmaterialien für den Zahnersatz bekannt, die aus einem Gemisch von Kunststoff, Füllern und Farbstoff bestehen. Die Füller sollen die Schrumpfung bei der Polymerisation verringern und einige mechanischen Eigenschaften verbessern. Komposite brechen leicht.

Außerdem sind ganze vorgefertigte Ersatzzähne auf Faserverbundbasis bekannt (DE-PS 32 43 861), die kurzfaserige Kohlenstoffasern enthalten. Die kurzen Fasern können die Kräfte über den geforderten Raum bzw. Fläche nicht aufnehmen, deshalb neigt auch dieses Material zu brechen. Auch dieser Zahnersatz besitzt nicht die geforderten Eigenschaften. Kohlenstoffaser ist wegen ihrer Farbe aus ästhetischen Gründen kaum akzeptabel.

Aus der US-A-5 176 951 ist ein Verfahren zur Verstärkung von Zahnprothesen bekannt, bei dem ein Gewebe an die zu verstärkende Stelle nach dem Aufbringen einer Schicht eines Dentalwerkstoffs aufgelegt wird und anschließend mit einer weiteren Schicht des Dentalwerkstoffs bedeckt wird. Bei diesem Verfahren wird ein spezielles Gewebe verwendet, das mit einer Spezialschere geschnitten werden muß, nicht mit bloßen Händen berührt werden darf und vor dem Auflegen mit Monomerflüssigkeit getränkt werden muß. Dieses Verfahren ist daher sehr umständlich, zeitaufwendig und störanfällig und vor allen Dingen in seinen Anwendungen sehr beschränkt. Insbesondere eignet sich dieses Verfahren nicht für die Herstellung von Zahnfüllungen.

Aus der JP-A 97625/1993 ist ein photopolymerisierbares Dentalmaterial zur Herstellung einer Krone bekannt, das erhalten wird durch Ausformung eines Gäserverstärkten Kunststoffs in umgehörtetem Zustand in eine Röhrenform. Der Kunststoff kann Aerosil oder Quarzpulver enthalten.

Aus der US-A 5,098,304 ist ein Dentalmaterial bekannt, das ein Glasfasermaterial enthält, das mit einem photopolymerisierbaren Komposit zu einem Splint verarbeitet werden kann.

Aus der DE 38 19 777 sind füllstoffhaltige, photopolymerisierbaren Massen mit kurzen faserförmigen Füllstoffen bekannt.

Die EP-B1 0726 739 offenbart ein vorimprägniertes Gewebestud für die Herstellung einer dentalen Prothese.

Aus der US-A 5,218, 070 sind lagerstabile, photopolymerisierbare Dentalzusammenschungen in einem Behälter bekannt.

Es ist daher Aufgabe der Erfindung einen Dentalwerkstoff bereitzustellen, der einfach, schnell und störungsfrei zu verarbeiten ist, in seiner Anwendungsbreite keinen Beschränkungen unterliegt, also insbesondere zur Herstellung von Zahnfüllungen geeignet ist und dennoch die Vorzüge einer Faserverstärkung optimal zur Entfaltung bringt.

Diese Aufgabe wird gemäß Anspruch 1 gelöst. Weitere vorteilhafte Ausgestaltungen sind Gegenstand von Unteransprüchen. Dadurch, daß neben dem Gewebe band noch ein mikrofeiner Füllstoff in der Matrix vorliegt hat das gebrauchsfertig vorliegende Material einerseits die richtige Konsistenz für eine stabile Lagerung und andererseits die richtige Zusammensetzung für die Verwirklichung einer idealen Kombination von mechanischen Eigenschaften, z.B. bezüglich Zug-, Biege- und Schlagfestigkeit einerseits und Druck- und Abriebfestigkeit andererseits. Durch die Erfindung wird ein bisher nicht erkanntes Potential der Photopolymerisation von Dentalwerkstoffen erschlossen.

Damit werden einerseits bisher schon verwirklichbare Anwendungen grundlegend verbessert, z. B. hinsichtlich der Kombination von Schlagfestigkeit, Zugfestigkeit, Druckfestigkeit, und Abriebfestigkeit. Andererseits werden völlig neuartige Anwendungsgebiete erschlossen, z. B. ein Kronenaufbau mit in Umfangsrichtung orientierten Verstärkungsfäden bei dem Röntgentransparenz vorliegt, oder eine röntgentransparente Maryland-Brücke ohne Metallgerüst. Ferner können extrem formstabile Zahnfüllungen erstellt werden, bei denen die Ausbildung eines Randspaltes vor, während oder nach der Polymerisation vollkommen unterbunden werden kann. Dabei kann vorteilhafterweise die gesamte Kavität in einem einzigen Arbeitszyklus gefüllt und die Füllung polymerisiert werden, sofern Fasermaterial mit guten Lichtleitungseigenschaften verwendet wird. Ein zeitaufwendiger, schichtweiser Aufbau, für den Patienten unangenehm und für den Zahnarzt kostspielig, kann daher unterbleiben. Vor allem aber wird bei dieser außerordentlichen Diversität der erschlossenen Anwendungsgebiete eine bisher nicht denkbare Vereinheitlichung des Dentalmaterials erreicht. Mit einem einzigen Dentalwerktoff-Typ können nun verschiedene Dentalarbeiten ausgeführt werden. Dies hat große Vorteile hinsichtlich der Ausbildung und der handwerklichen Fähigkeiten des Fachpersonals, dem ein solches Material in die Hand gegeben wird. Mit der Erfindung wird somit eine Umwälzung der Dentaltechnik auf Kunststoffbasis eingeleitet.

Die werden in eine Matrix gebettet, die aus Kunststoffen wie Duro- oder Thermoplasten bestehen.

Die Gewebebänder sind mit an sich bekannten mit Licht oder UV-Strahlen härtbaren und/oder polymerisierbaren Dentalmassen vorimprägniert. Besonders geeignet sind Compomermassen. Diese Matrixkunststoffe enthalten zusätzlich mikrofeine, anorganische Füllstoffe. Weiterhin können auch andere übliche anorganische oder organische Füllstoffe verwendet werden. Vorzugsweise können 5-80 Gew.-% und speziell 10-60 Gew.-% in der Dentalmasse (ohne Gewebe oder Fasern) vorliegen.

Als Fasermaterial kommen sowohl anorganische Fasern, wie Glasfasern, Keramikfasern usw. als auch organische Fasern, wie Polyethylenfasern, Polyesterfasern usw. in Frage. Wird ein Thermoplast als Matrix verwendet, sollten nur nichtschmelzende Fasern eingebaut werden.

Da Fasern nur Zugkräfte aufnehmen können, werden diese vorzugsweise so verlegt, daß sie auf Zug beansprucht werden. Druckkräfte auf die Matrix werden nach Möglichkeit so umgeleitet, daß diese als Zugkräfte von den Fasern aufgenommen werden. Dies erreicht man durch Verlegen der im wesentlichen in geschlossenen Bahnen, Ringen und/oder Knäueln in der Matrix. Der Anteil des Fasermaterials soll möglichst hoch sein, und zwar soll dieser zwischen 20% und 90% liegen. In ausgeführten Probegegenständen hat sich ein Wert von annähernd 70% gut bewährt. Durch den oben erwähnten Anteil an Fasern wird die gefürchtete Schrumpfung beim Aushärten stark reduziert. Auch gegen Abrieb sollen die gewählten Fasern eine hohe, Verschleißfestigkeit aufweisen.

Das Fasermaterial für Füllungen und Inlays/Onlays besteht aus Gewebebändern, die mit verschiedenen Kunststoffen vorimprägniert sind. Als Matrixkunststoffe eignen sich Licht-, UV- bzw. Laser-Strahlen-härtende Materialien. Diese Materialien können schon teilweise polymerisiert sein, wodurch die Nachpolymerisierzeit im Mund und die Schrumpfung reduziert wird. Das Matrixmaterial soll gewisse viskoelastische Eigenschaften haben, um Rißbildung und dadurch Leckagen zu vermeiden.

Zunächst wird ein Verfahren beschrieben, bei dem eine Füllung direkt im Mund des Patienten hergestellt wird.

In die präparierte Kavität im Zahn wird beispielsweise ein UV-strahlenhärtendes, vorimprägniertes Band gewebe geforderter Breite zickzack- oder ringförmig gelegt und fest gestopft bis das Loch gut gefüllt ist. Der Kunststoff sollte leicht klebrig sein, so daß die Schichten aneinander haften. Mit einem Licht-, UV- bzw.Laserstrahen-durchlässigen Material (Stampfer) wird Druck auf die Füllung ausgeübt und gleichzeitig wird die Füllung zum Polymerisieren mit Licht-, UV- bzw. Laser-Strahlen bestrahlt. Die noch plastische Füllung wird während des Polymerisierens gegen die Zahnwände gepresst, so daß der gefürchtete Spalt zwischen Zahn und Füllung nicht entstehen kann. Nach dem Aushärten kann die Okklusionsfläche mit herkömmlichen zahnärztlichen Werkzeugen nachgearbeitet werden. So eine Füllung ist überraschend einfach und schnell, somit billig zu installieren, außerdem ist sie von hervorragender Qualität.

Als Stampfer zur Ausübung von Druck auf die Füllung während der Polymerisation kann jedes Licht-, UV- bzw. Laserstrahlen-durchlässige Material verwendet werden. Bevorzugt ist ein Material, das elastische Eigenschaften hat, die eine im wesentlichen gleichmäßige Druckverteilung auf die Oberfläche der Füllung erlauben. Geschäumte Kunststoffmaterialien haben sich zu diesem Zweck besonders bewährt. Die Druckfläche des Stampfers ist vorzugsweise mindenstens so groß wie die Okklusionsfläche der Füllung. Als besonders vorteilhaft hat sich eine Vorrichtung erwiesen, die auf die Spitze einer Lichtpistole gesteckt werden kann. Dadurch ist es möglich mit der Spitze der Lichtpistole während der Polymerisation Druck auf die Füllung auszuüben, ohne daß dabei die Optik der Lichtpistole durch das Füllungsmaterial verklebt wird.

Das Licht-, UV- bzw. Laser-Strahlen-härtende Material muß vor der Verarbeitung in einem vor Licht- bzw. UV-Strahlen sicheren, dunklen Behälter aufbewahrt werden. Es handelt sich beispielsweise um vorimprägnierte endlose Gewebebänder, die stückweise aus dem Behälter gezogen und in geforderter Länge abgeschnitten werden können. Das Material ist ohne weitere Vorarbeiten sofort einsetzbar. Es können auch diskrete Bandabschnitte in einer Hüllfolie oder in einem Kunststoffbehälter untergebracht sein.

Zur preiswerten Herstellung von Inlays/Onlays kann ein sehr einfaches Verfahren angewandt werden.
Zunächst füllt man die Kavität im Zahn mit einem an sich bekannten lichthärtenden, leichtelastischen Material und modelliert die Füllung sehr genau passend und zwar auch in der Okklusionsfläche, wie das bei Herstellung von provisorischen Füllungen bekannt ist. Dann härtet man die Masse mit Licht aus und nimmt die elastische Füllung aus der Kavität im Zahn. In einer vorgefertigten Form wird der elastische positive Abdruck so teilweise beispielsweise in Gips gegossen, daß man ihn später problemlos herausnehmen kann. Das bedeutet, daß ein Teil des provisorischen Inlays frei liegt. Die Trennfläche, die zur eindeutigen Zuordnung besondere Passformen hat, wird mit einem Trennmittel versehen, und anschließend wird darauf eine obere Gipsform gegossen. Die beiden Formhälften werden nach dem Aushärten getrennt, und der elastische Abdruck entnommen. Die entstandene Kavität entspricht genau der Form des herzustellenden Inlays/Onlays. Die Kavität im Gips wird wie oben beschrieben mit beispielsweise mit Duroplast stark vorimprägniertem Gewebe gut gefüllt. Jetzt wird die genau passende obere Formhälfte auf die untere Formhälfte gepresst.

Über einen Abflußkanal kann überschüssiger Kunststoff abfließen. Das in der Kavität entstehende Inlay/Onlay erhält eine hohe Passgenauigkeit. Wenn der Kunststoff ausgehärtet ist, wird die Form geöffnet, und das Inlay/Onlay kann entnommen werden. Bei Hinterschneidungen muß die untere Gipsform zerstört werden.

Das Positivmodell kann auch aus Wachs gefertigt sein. Dieses Modell läßt sich leicht durch Erwärmen des Wachses aus der Form entfernen.
Als Matrix kann auch hier ein lichthärtender Kunststoff verwendet werden. Für diesen Fall muß bei der oberen Formhälfte eine größere Öffnung vorgesehen sein, in die beim Vergießen zur Formherstellung ein lichtdurchlässiger Kunstharz eingebracht wird.

Solche Inlays/Onlays können auch mit thermoplastischen Kunststoffen hergestellt werden. Das Gewebe sollte dann möglichst aus einem nichtschmelzendem Material bestehen.

Auch Kronen und Brücken können vorteilhaft auf die vorher beschriebene Art mit faserverstärkten Kunststoffen hergestellt werden. Es können z.B. sehr dünnwandige Kronen gefertigt werden, die bisher fast nur in Gold hergestellt wurden. Oder man fertigt zur Herstellung von Inlays/Onlays, Kronen und Brücken in bekannter Weise Abdrücke und Meistermodelle. Am Meistermodell werden - wie im Mund - aus elastischem Material positive Inlays/Onlays, Kronen und Brücken hergestellt. Dann werden wie vorher beschrieben die Artikel in Gipsformen hergestellt. Am Meistermodell kann auch mit Wachs modelliert werden. Die fertigen Artikel können am Meistermodell angepaßt und nachgearbeitet werden.

Es können auch direkt am Meistermodell Inlays/Onlay usw. aus vorimprägniertem Gewebe gefertigt werden. Beispielsweise zur Herstellung einer dünnwandigen Krone wickelt man zweckmäßigerweise ein vorimprägniertes Gewebeband um den Zahnstumpf des Meistermodells bis die Stärke des unteren Zahnstumpfes erreicht ist. Dann legt man in bekannter Weise eine Matrize um den Zahnstumpf. Man füllt die Matrize anschließend voll aus und drückt den Abdruck der Gegenzähne auf die Anordnung.

Eventuell legt man eine Zwischeneinlage ein, die die Vertiefungen simulieren. Es entsteht eine passgenaue Krone, die nur noch in der Kaufläche nachgearbeitet werden muß.

Es ist einfach und vorteilhaft, mit einer thermoplastischen Matrix im Gewebe am Meistermodell einen Zahnersatz herzustellen. Bei Zahnersatzgegenständen wie Inlays/Onlays, Kronen, künstlichen Zähnen, Brücken usw. ist es preiswert und technisch günstig, erfindungsgemäß vorgefertigte Teile zu verwenden, die dann nur noch zusammengesetzt und bzw. oder nachgearbeitet werden müssen. Serienmäßig vorgefertigte Teile können mit immer gleicher, garantierbar hoher Qualität kontrolliert hergestellt werden.
Bei einem Zahnschaden kann man aus einem Sortiment von erfindungsgemäß vorgefertigten Zahnersatzteilen das annähernd passende Teil z.B. einen Zahn oder die zueinander passenden Teile heraussuchen und dann durch Abtragen von Material mit Automaten und/oder Standardwerkzeugen den speziellen Erfordernissen anpassen. Beim Arbeiten mit Automaten wird die Außenkontur des geschädigten Zahnes vermessen und danach der Zahnersatz hergestellt. Bisher wurde der Ersatz meist aus Keramikblöcken geschliffen. Bei dem erfindungsgemäßen Zahnersatz kann beispielsweise zur Herstellung einer Krone ein vorgefertigter Zahn durch Materialabtrag den Erfordernissen angepasst werden. Das gilt auch für Inlays/Onlays.

Zur Herstellung einer Brücke können drei oder mehrere nebeneinander zusammenhängende mit durchgehenden Fasern vorgefertigte Zähne verwendet werden. Eine der vielen Möglichkeiten, eine Zahnlücke zu überbrücken, sei kurz beschrieben: Es fehle ein Zahn und die beiden Nachbarzähne seien einwandfrei. Man nimmt ein vorgefertigtes Teil, das aus einem Zahn, der in der Tiefe größer als ein normaler Zahn ist, und aus an beiden Seiten hinten integrierten Fahnen besteht, die mittels in erfindungsgemäßer Form angeordneten Geweben verbunden sind. Am Abdruck können Zahn und Fahnen dann derart zugefräst werden, daß die Fahnen genau passend hinten an den Nachbarzähnen angeklebt werden können. Eine analoge Technik kann man in Kombination mit Inlays/Onlays mit den Nachbarzähnen anwenden.

Solche Brücken kann man natürlich auch mit den vorher beschriebenen Methoden z.B. in Gipsformen vorteilhaft herstellen.

Die Klebeflächen des Zahnersatzes sollen eine gewisse Rauhigkeit aufweisen, damit eine haltbare Klebung am Zahn erfolgen kann. Es kann zweckmäßig sein, den Zahnersatz in mehreren Schichten aufzubauen, wobei die Matrix in den unterschiedlichen Schichten unterschiedliche Härte haben kann. Da das Fasermaterial und der Kunststoff mit dem menschlichen Gewebe verträglich ist, lassen sich auch erfindungsgemäße Implantate herstellen. Eine feste Bindung zwischen Fasern und Matrix sollte unbedingt vorhanden sein. Die Farbgebung kann durch Einfärben des Kunststoffes und/oder Einfärben der Fasern geschehen.

Das vorimprägnierte Gewebeband kann in verschiedenen Verfahren hergestellt und verpackt werden. Das Gewebe wird durch ein Bad mit Kunstharz über Rollen gezogen und so getränkt. Die Rollen sorgen für eine totale Durchtränkung und ein Ausdrücken eventueller Lufteinschlüsse. Eine kurze Nachbehandlung kann erforderlich sein, damit die Matrix zäh wird. Das vorimprägnierte Gewebe kann meanderförmig in die Behälter eingelegt werden.

## Patentansprüche

1. Gebrauchsfertig verpackter, mit UV-Strahlen, Laserstrahlen oder Licht härtbarer Dentalwerkstoff für die Herstellung von Zahnfüllungen, Inlays, Onlays, Kronen, Brücken, künstlichen Zähnen, Stiftzähnen, Zahnprothesen und Zahnimplantaten mit
(a) einem für UV-oder Licht undurchlässigen Behälter, in dem
(b) ein als vorimprägniertes Gewebeband vorliegender Werkstoff so untergebracht ist, dass er bei Lagerung vor der Einwirkung von UV- oder Lichtstrahlen geschützt ist und bei Gebrauch entnehmbar ist, wobei der Werkstoff folgendes umfasst:
(b1) eine mit UV-Strahlen, Laserstrahlen oder Licht zu einem duroplastischen oder thermoplastischen Dentalkunststoff polymerisierbare und/oder härtbare Matrix, der
(b2) mikrofeiner, anorganischer Füllstoff einverleibt ist und in die
(b3) ein Gewebe in Bandform eingebettet ist.

2. Dentalwerkstoff nach Anspruch 1, **dadurch gekennzeichnet, dass** das Gewebe aus anorganischem oder organischem Material besteht.

3. Dentalwerkstoff nach Anspruch 2, **dadurch gekennzeichnet, dass** das anorganische Material Glas oder Keramik ist.

4. Dentalwerkstoff nach Anspruch 2, **dadurch gekennzeichnet, dass** das organische Material Polyethylen oder Polyester ist.

5. Dentalwerkstoff nach Anspruch 1, **dadurch gekennzeichnet, dass** das Gewebe in Form geschlossener Bahnen, wie Ringen, Meanderbahnen oder Knäueln vorliegt.

6. Dentalwerkstoff nach Anspruch 1, **dadurch gekennzeichnet, daß** der Anteil des Gewebes (b3) im Bereich von 20 bis 90% liegt.

7. Dentalwerkstoff nach einem der Ansprüche 1 bis 6, **dadurch gekennzeichnet, dass** der lichtundurchlässige Behälter als Spender für einen gewickelten, bandförmigen Werkstoff ausgebildet ist, oder als Kunststoffbehälter oder Hüllfolie für diskrete Bandabschnitte des Werkstoffs vorliegt.

## Claims

1. Ready-to-use, packed dental material that can be hardened by UV-rays, laser beams or light for producing tooth fillings, inlays, onlays, crowns, bridges, artificial teeth, post crowns, tooth prosthetics and tooth implants with
a) a container that is impermeable to UV or light in which
b) a material existing as a preimpregnated fabric strip is stored in such a way that it is protected during storage from the effect of UV or light rays and can be removed on use, wherein the material comprises the following:
b1) a matrix that can be polymerised and/or hardened by UV rays, laser beams or light to form a thermoset or thermoplastic dental plastic, which
b2) incorporates microfine, inorganic filler material and in which
b3) a fabric in strip form is embedded.

2. Dental material according to claim 1, **characterised in that** the fabric consists of inorganic or organic material.

3. Dental material according to claim 2, **characterised in that** the inorganic material is glass or ceramic.

4. Dental material according to claim 2, **characterised in that** the organic material is polyethylene or polyester..

5. Dental material according to claim 1, **characterised in that** the fabric is in the form of closed webs such as rings, meandering webs or coils.

6. Dental material according to claim 1, **characterised in that** the fabric content (b3) is in the range of 20 to 90 %.

7. Dental material according to any of claims 1 to 6, **characterised in that** the light impermeable container is designed as a dispenser for a coiled strip-like material, or as a plastic container or covering film for discrete strip sections of the material.

## Revendications

1. Matériau dentaire, emballé, prêt à l'emploi et durcissable par des rayons ultraviolets, par des rayons laser ou par la lumière, destiné à la fabrication de plombages de dents, d'inlays, d'onlays, de couronnes, de bridges, de fausses dents, de dents sur pivot, de prothèses et d'implants dentaires, avec
(a) un récipient opaque aux rayons ultraviolets ou à la lumière, dans lequel
(b) est logé un matériau de telle manière qu'il soit protégé, lors de l'entreposage, contre les effets des rayons ultraviolets ou des rayons lumineux et qu'il puisse être extrait de la boîte lors de l'utilisation, le matériau comprenant les éléments suivants :
(b1) une matrice propre à être polymérisée et/ou durcie par des rayons ultraviolets, par des rayons laser ou par la lumière pour donner une matière artificielle à usage dentaire, thermodurcissable ou thermoplastique, matrice
(b2) à laquelle est associée une matière de remplissage inorganique et microfine,
(b3) et dans laquelle est noyé un tissu sous forme de nappe, de bande, de tube flexible ou de cordon, ou un faisceau de fibres sans fin.

2. Matériau dentaire selon la revendication 1, **caractérisé en ce que** le tissu ou les fibres sans fin consiste(nt) en une matière inorganique ou organique.

3. Matériau dentaire selon la revendication 2, **caractérisé en ce que** la matière inorganique est du verre ou de la céramique.

4. Matériau dentaire selon la revendication 2, **caractérisé en ce que** la matière organique est du polyéthylène ou du polyester.

5. Matériau dentaire selon la revendication 1, **caractérisé en ce que** le tissu ou le faisceau de fibres sans fin se présente sous la forme de bandes fermées, par exemple des anneaux, des bandes en méandres ou des pelotes.

6. Matériau dentaire selon la revendication 1, **caractérisé en ce que** la proportion du tissu ou des fibres sans fin (b3) est comprise dans la fourchette de 20 à 90 %.

7. Matériau dentaire selon l'une des revendications 1 à 6, **caractérisé en ce que** le récipient opaque à la lumière est réalisé sous la forme d'un distributeur pour un matériau en forme de bande et enroulé, ou bien en tant que récipient en matière plastique ou en tant que film d'emballage pour des longueurs discrètes de bandes du matériau.
